# EUROPEAN PATENT APPLICATION

(11) **EP 4 498 285 A1**
(43) Date of publication of application: **29.01.2025**
(21) Application number: 22933312.5
(22) Date of filing: 23.03.2022
(51) Int. Cl.: G06N 3/08, G06N 20/00

(54) **MAP GENERATION DEVICE, MAP GENERATION METHOD, AND NON-TRANSITORY COMPUTER-READABLE MEDIUM**

(71) Applicant: NEC Corporation, 108-8001 Tokyo (JP); Tohoku University, Sendai-shi, Miyagi 980-8577 (JP)
(72) Inventor: KUWAMORI Naoki, Tokyo 108-8001 (JP); MUSA Akihiro, Tokyo 108-8001 (JP); SATOU Yoshihiko, Tokyo 136-8627 (JP); KOBAYASHI Hiroaki, Sendai-shi, Miyagi 980-8577 (JP); KIKUGAWA Gota, Sendai-shi, Miyagi 980-8577 (JP); OKABE Tomonaga, Sendai-shi, Miyagi 980-8577 (JP); KOMATSU Kazuhiko, Sendai-shi, Miyagi 980-8577 (JP); KAWAGOE Yoshiaki, Sendai-shi, Miyagi 980-8577 (JP)
(74) Representative: Betten & Resch
(86) International application number: PCT/JP2022/013480
(87) International publication number: WO 2023/181167

(57) **Abstract**

A map generation apparatus (2000) acquires a plurality of pieces of material specification information (10) and physical property information (20). The material specification information (10) indicates material specification. The physical property information (20) indicates a physical property value of each of a plurality of physical properties for a product (70) that can be generated with the material specification indicated by the corresponding material specification information (10). The map generation apparatus (2000) clusters the plurality of pieces of physical property information (20) and extracts some pieces of physical property information (20) from each cluster. The map generation apparatus (2000) generates a self-organizing map (30) by using the extracted physical property information (20). In the self-organizing map (30), a physical property vector indicating a value related to a physical property value of each of a plurality of types of physical properties of the product (70) is assigned to each node on a map space. The map generation apparatus (2000) assigns the material specification information (10) corresponding to the physical property information (20) extracted from the cluster to any one node of the self-organizing map (30).

## Description

### Technical Field

The present disclosure relates to a technique for providing information related to product development.

### Background Art

In product development, it is useful to ascertain a relationship between a material and a product. Therefore, a system that supports ascertaining of a relationship between a material and a product has been developed. For example, Patent Literature 1 discloses a system that supports ascertaining of a causal relationship between a design value of a tire and a physical property value by using a self-organizing map.

### Citation List

### Patent Literature

Patent Literature 1: Japanese Unexamined Patent Application Publication No. 2016-148988

### Summary of Invention

### Technical Problem

In Patent Literature 1, a self-organizing map is used to determine which variable is an important factor among a plurality of design variables of a tire. Therefore, it is not assumed that the self-organizing map is used for other purposes. The present disclosure has been made in view of such a problem, and an objective of the present disclosure is to provide a new technique for providing information useful for product development.

### Solution to Problem

According to the present disclosure, a map generation apparatus includes: acquisition means for acquiring a plurality of pieces of material specification information indicating a material specification, and acquiring physical property information indicating a physical property value of each of a plurality of physical properties of a product that can be generated with the material specification indicated by the material specification information for each piece of the material specification information; clustering execution means for clustering a plurality of pieces of the physical property information; generation means for extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and assignment means for assigning the material specification information corresponding to the extracted physical property information to one of the nodes.

According to the present disclosure, a map generation method is executed by a computer. The map generation method includes: an acquisition step of acquiring a plurality of pieces of material specification information indicating a material specification, and acquiring physical property information indicating a physical property value of each of a plurality of physical properties of a product that can be generated with the material specification indicated by the material specification information for each piece of the material specification information; a clustering execution step of clustering a plurality of pieces of the physical property information; a generation step of extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and an assignment step of assigning the material specification information corresponding to the extracted physical property information to one of the nodes.

According to the present disclosure, a non-transitory computer readable medium stores a program for causing a computer to execute the map generation method of the present disclosure.

### Advantageous Effects of Invention

According to the present disclosure, a new technique for providing information useful for product development is provided.

### Brief Description of Drawings

Fig. 1 is a diagram illustrating an overview of an operation of a map generation apparatus in a first example embodiment.
Fig. 2 is a block diagram illustrating a functional configuration of the map generation apparatus in the first example embodiment.
Fig. 3 is a block diagram illustrating a hardware configuration of a computer that implements the map generation apparatus in the first example embodiment.
Fig. 4 is a flowchart illustrating a flow of processing executed by the map generation apparatus in the first example embodiment.
Fig. 5 is a diagram illustrating material specification information in a table format.
Fig. 6 is a diagram illustrating physical property information in a table format.
Fig. 7 is a diagram illustrating a structure of a self-organizing map in a table format.
Fig. 8 is a diagram illustrating a structure of the self-organizing map to which material specification information is assigned.
Fig. 9 is a diagram illustrating a map image.

### Example Embodiment

Hereinafter, an example embodiment of the present disclosure is described in detail with reference to the drawings. In the drawings, the same or corresponding elements are denoted by the same reference numerals, and repeated description is omitted as necessary for clarity of description. In addition, unless otherwise described, predetermined information such as predetermined values and threshold values are stored in advance in a storage device or the like accessible from a device using the information.

### [First Example Embodiment]

### <Overview>

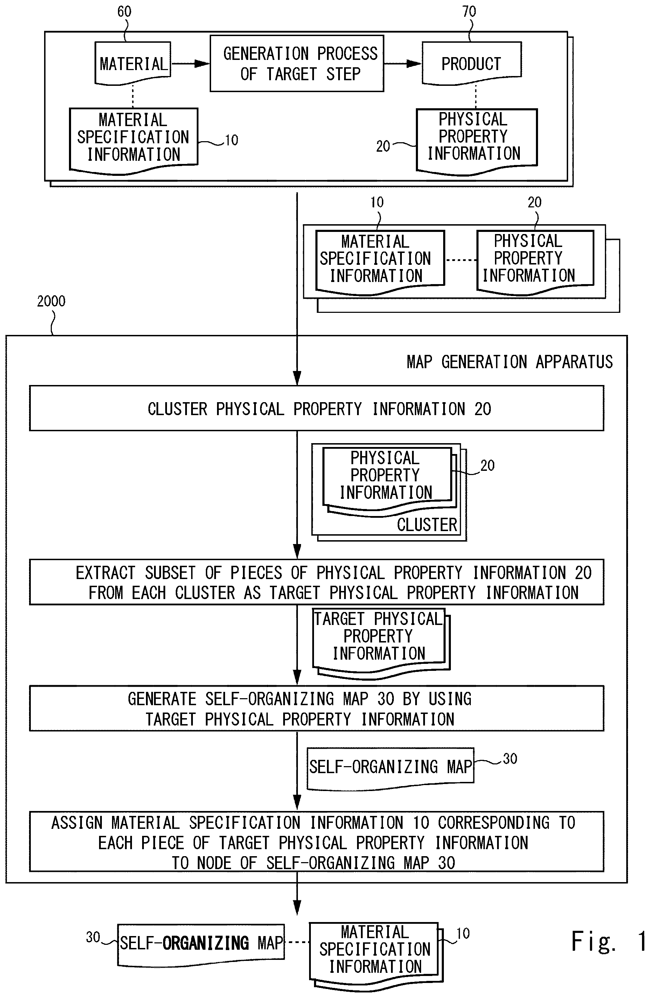

Fig. 1 is a diagram illustrating an overview of an operation of a map generation apparatus 2000 according to a first example embodiment. Here, Fig. 1 is a diagram for facilitating understanding of the overview of the map generation apparatus 2000, and the operation of the map generation apparatus 2000 is not limited to that illustrated in Fig. 1.

The map generation apparatus 2000 generates a self-organizing map 30 representing a distribution of physical properties of various products 70 that can be generated in a specific step (hereinafter, a target step) of product development. The product 70 is predicted to be produced by processing a material 60 in a production process of the target step, or is actually produced. The material 60 is a material used for generating the product 70. The material 60 of various patterns may be used in the target step. The physical property of the product 70 can vary depending on the material 60 to be used.

A pattern of the material 60 is identified by a material specification. In other words, the materials 60 having different material specifications are treated as the materials 60 of different patterns. On the other hand, the materials 60 having the same material specification are treated as the materials 60 of the same pattern.

The material specification is represented by, for example, the type of material, the types of substances constituting the material, the blending ratio between the respective substances, the type of processing performed to create the material, and the like. Examples of the type of material include carbon fiber reinforced plastic and stainless steel. For example, it is assumed that the material 60 is carbon fiber reinforced plastic. In this case, the material specification of the material 60 include the type (polyacrylonitrile fiber, cellulose carbide fiber, or the like) of each of one or a plurality of carbon fibers constituting the material 60, the type (epoxy, polyether terephthalate, or the like) of each of one or a plurality of resins constituting the material 60, and the blending ratio of these substances. In addition, the material specification may further include the type of fiber direction polymerization method, the type of pressure bonding method, resin composition, and the like.

Note that the target step may be a single step or a combination of a plurality of continuous steps. In this case, the product 70 is a product that can be generated by processing the material 60 in the plurality of continuous steps. For example, it is assumed that the target step is a combination of a step P1 and a step P2. In this case, the product 70 is obtained by processing the material 60 in a production process of the step P1 and then processing the product of the step P1 in the production process of the step P2.

The self-organizing map 30 has a plurality of nodes disposed on an m-dimensional map space. Here, m is set to 2 or 3 so that the map space can be visually expressed (for example, in an image). In the visually expressed map space, each node is represented by, for example, a square of a grid or a vertex of a lattice.

Multi-dimensional data representing the magnitude of a physical property value of each of a plurality of types of physical properties (hereinafter, a physical property vector) is assigned to each node of the self-organizing map 30. For example, it is assumed that four types of physical properties such as flame retardancy, heat resistance, elastic modulus, and toughness are used. In this case, the physical property vector is four-dimensional data representing the magnitude of the physical property value of each of these four types of physical properties. Hereinafter, the number of dimensions of the physical property vector is set to n. Here, n > m. That is, in the self-organizing map 30, a space of the physical property vector is a high-dimensional space, and the map space is a low-dimensional space.

In order to generate such a self-organizing map 30, the map generation apparatus 2000 acquires the material specification information 10 indicating the material specification for each of the plurality of patterns of the material 60 (in other words, the material 60 identified by each of the plurality of patterns of the material specification), and the physical property information 20 corresponding to the material specification information 10 for each of the plurality of patterns of the material 60. That is, the map generation apparatus 2000 acquires a plurality of pairs of the material specification information 10 and the physical property information 20. The physical property information 20 corresponding to the material specification information 10 indicates the physical property value for each of the plurality of types of physical properties for the product 70 that can be generated in the target step by using the material 60 of the material specification represented by the material specification information 10. The type of physical properties is, for example, flame retardancy, heat resistance, elastic modulus, toughness, or the like. Note that the number of types of physical properties indicated by the physical property information 20 is equal to or larger than the number n of dimensions of the physical property vector.

The map generation apparatus 2000 performs clustering on a plurality of pieces of acquired physical property information 20 based on the physical property value indicated by each piece of physical property information 20. As a result, a plurality of clusters of the physical property information 20 are generated.

The map generation apparatus 2000 extracts, from each cluster, a subset of the plurality of pieces of physical property information 20 included in the cluster. Each piece of physical property information extracted here is referred to as "target physical property information". The map generation apparatus 2000 performs training of the self-organizing map 30 by using the physical property value indicated by each piece of target physical property information to determine the physical property vector to be assigned to each node, thereby generating the self-organizing map 30. Therefore, the map generation apparatus 2000 generates the self-organizing map 30 by using not all the pieces of the acquired physical property information 20 but only a subset of the pieces of the physical property information 20. The physical property information 20 used for generating the self-organizing map 30 is determined based on the result of clustering.

Further, for each piece of target physical property information, the map generation apparatus 2000 assigns the material specification information 10 corresponding to the target physical property information to one of the nodes of the self-organizing map 30. Specifically, the map generation apparatus 2000 assigns the material specification information 10 corresponding to the target physical property information to the node having the physical property vector that is most similar to the n-dimensional data obtained from the target physical property information. As a result, each pair of the target physical property information and the material specification information 10 is associated with one of the nodes of the self-organizing map 30. That is, the material specification is associated with the physical properties of the product 70 in the self-organizing map 30.

### <Example of Advantageous Effect>

In product development, in order to generate a product having desired physical properties, a search for a material capable of generating such a product may be performed. As a method of achieving such a search, there is a method of simulating generation of a product or experimentally generating a product while variously changing material specifications. Furthermore, a method of performing inverse analysis of predicting material specification from preferable physical properties by using a correspondence between material specification and physical properties of a product, which are accumulated through such simulations and experimental generations of the products (correspondence between the material specification information 10 and the physical property information 20 in the present disclosure) is also considered.

In this regard, the map generation apparatus 2000 generates the self-organizing map 30 by using the physical property vector obtained from each of the plurality of pieces of physical property information 20, and assigns the material specification information 10 indicating the material specification to each node of the self-organizing map 30. By using such a self-organizing map 30, it is possible to ascertain the distribution of material specifications on the self-organizing map representing the distribution of physical properties. That is, by using the self-organizing map 30, it is possible to ascertain the correspondence between the distribution of physical properties and the distribution of material specifications. Thus, the self-organizing map 30 can be used for the inverse analysis described above.

In addition, when simulation or the like is performed while variously changing the material specification, physical properties of the obtained product may be biased. For example, even if simulation or the like is performed while randomly changing the pattern of the material specification to be uniformly distributed, physical properties of the product are not necessarily uniformly distributed. Thus, among the plurality of pieces of physical property information 20 obtained by simulation or the like, there may be many pieces of physical property information 20 indicating physical properties similar to each other.

In a case where there are many pieces of physical property information 20 indicating physical properties similar to each other, if the self-organizing map 30 is generated by using all the pieces of physical property information 20, the influence of the physical properties indicated by many pieces of physical property information 20 similar to each other becomes strong in the self-organizing map 30. Therefore, there is a possibility that it is not possible to represent the distributions of material specifications and physical properties with high accuracy by the self-organizing map 30.

In this regard, the map generation apparatus 2000 clusters the plurality of pieces of acquired physical property information 20, extracts a subset of the pieces of physical property information 20 from each cluster, and generates the self-organizing map 30. As described above, by extracting and using the representative physical property information 20 from each cluster, it is possible to reduce the distribution bias of the physical properties for the physical property information 20 used for generating the self-organizing map 30. Thus, it is possible to generate the self-organizing map 30 with high accuracy.

Generating the self-organizing map 30 by using the representative physical property information 20 is particularly useful in a case where the target step is a combination of a plurality of steps. For example, it is assumed that the target step includes two steps of a first step of generating plastic from a plastic material and a second step of generating a propeller from the plastic generated in the first step. In this case, the material specification information 10 indicates information regarding the material of the plastic, information regarding the structure of the plastic, information regarding a production method of the plastic, and the like. The physical property information 20 indicates information regarding the physical properties of the propeller generated in the second step.

Here, in order to be able to generate a propeller having desired properties, it is preferable that appropriate material specification in the first step can be ascertained so that an appropriate plastic suitable for the propeller can be generated. Therefore, for example, the person in charge of the first step performs simulation and the like of the first step and the second step while variously changing material specification (plastic material, production method, and the like) as inputs of the first step. As a result, the self-organizing map 30 in which the distribution of the material specifications (input of the first step) of the plastic and the distribution of the physical properties (output of the second step) of the propeller are associated with each other is generated.

At this time, as described above, it is preferable to generate the self-organizing map 30 by using not all pieces of the physical property information 20 obtained by simulation or the like but a subset of the pieces of representative physical property information 20. Here, as a method of selecting the physical property information 20 to be used for generating the self-organizing map 30, a method of the person in charge of the first step performing manual selection is also considered. However, the person in charge of the first step may not have sufficient knowledge about the second step necessary for appropriately performing this selection. For example, in a case where the first step and the second step are performed by different companies, knowledge about the second step held by the company that performs the second step may not be provided to the company that performs the first step. Therefore, it is considered that it is difficult for the person in charge of the first step to manually select the physical property information 20 to be used for generating the self-organizing map 30.

In this regard, according to the map generation apparatus 2000, the physical property information 20 to be used for generating the self-organizing map 30 is automatically selected based on the result of clustering the physical property information 20. Thus, even in a case where the person in charge of the step using the material specification information 10 as an input does not have sufficient knowledge about the subsequent step, it is possible to generate the self-organizing map 30 with high accuracy by using a plurality of pieces of physical property information 20 having a small bias.

Hereinafter, the map generation apparatus 2000 in the present example embodiment will be described in more detail.

### <Example of Functional Configuration>

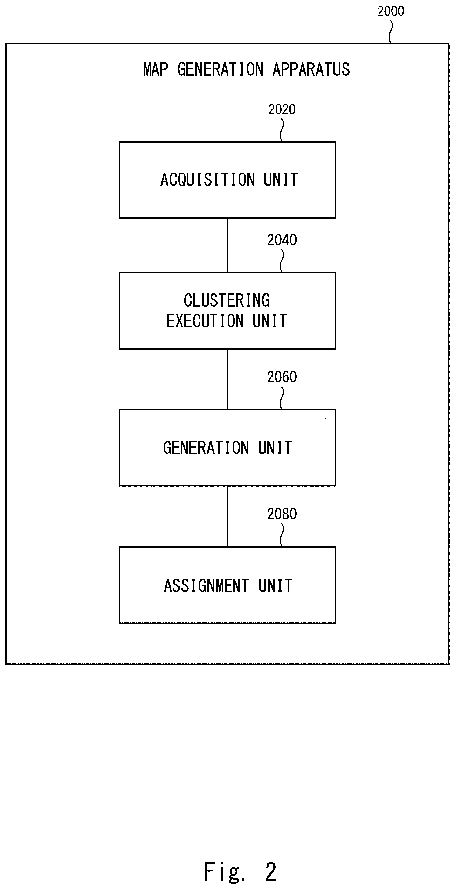

Fig. 2 is a block diagram illustrating a functional configuration of the map generation apparatus 2000 of the first example embodiment. The map generation apparatus 2000 includes an acquisition unit 2020, a clustering execution unit 2040, a generation unit 2060, and an assignment unit 2080. The acquisition unit 2020 acquires material specification information 10 and physical property information 20 for each of a plurality of patterns of the material 60. The clustering execution unit 2040 clusters the physical property information 20. The generation unit 2060 generates a self-organizing map 30 by using target physical property information. The target physical property information is the physical property information 20 extracted from each cluster. The subset of the plurality of pieces of physical property information 20 included in the cluster is extracted from each cluster. The assignment unit 2080 assigns the material specification information 10 corresponding to each piece of target physical property information to one of the nodes of the self-organizing map 30.

### <Example of Hardware Configuration>

Each functional configuration unit of the map generation apparatus 2000 may be implemented by hardware (for example, a hard-wired electronic circuit or the like) that implements each functional configuration unit, or may be implemented by a combination of hardware and software (for example, a combination of an electronic circuit and a program that controls the electronic circuit or the like). Hereinafter, a case where each functional configuration unit of the map generation apparatus 2000 is implemented by a combination of hardware and software will be further described.

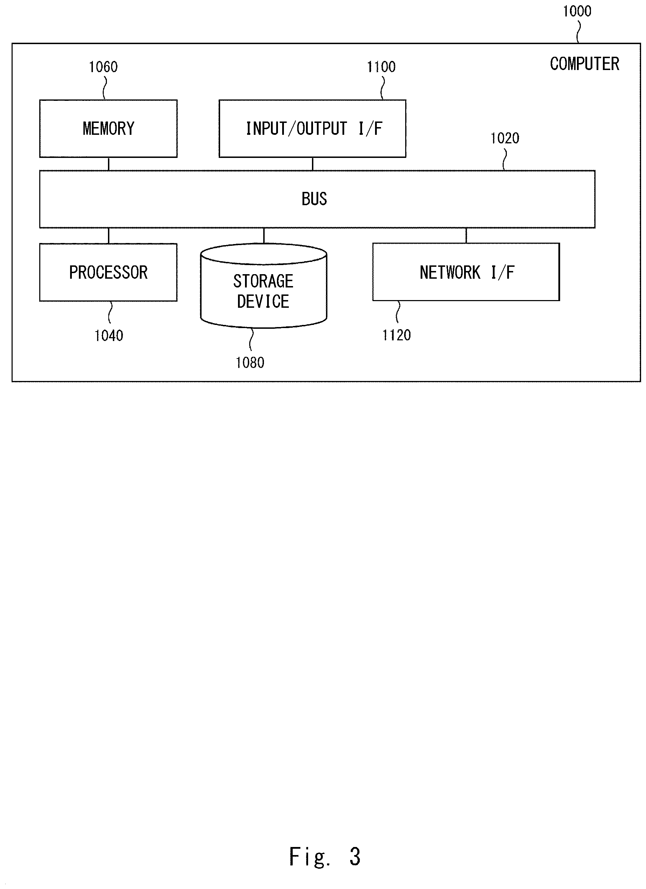

Fig. 3 is a block diagram illustrating a hardware configuration of a computer 1000 that implements the map generation apparatus 2000 of the first example embodiment. The computer 1000 is any computer. For example, the computer 1000 is a stationary computer such as a personal computer (PC) or a server machine. In another example, the computer 1000 is a portable computer such as a smartphone or a tablet terminal. The computer 1000 may be a specialpurpose computer designed to realize the map generation apparatus 2000, or may be a general-purpose computer.

For example, each function of the map generation apparatus 2000 is implemented in the computer 1000 by installing a predetermined application with respect to the computer 1000. The above-described application is configured with a program for implementing the functional configuration unit of the map generation apparatus 2000. Note that any method of acquiring the program is adopted. For example, the program can be acquired from a storage medium (a DVD disk, a USB memory, or the like) in which the program is stored. The program can also be acquired, for example, by downloading the program from a server machine that manages the storage device in which the program is stored.

The computer 1000 includes a bus 1020, a processor 1040, a memory 1060, a storage device 1080, an input/output interface 1100, and a network interface 1120. The bus 1020 is a data transmission path for the processor 1040, the memory 1060, the storage device 1080, the input/output interface 1100, and the network interface 1120 to transmit and receive data to and from each other. However, the method of connecting the processor 1040 and the like to each other is not limited to the bus connection.

The processor 1040 is any of processors such as a central processing unit (CPU), a graphics processing unit (GPU), or a field-programmable gate array (FPGA). The memory 1060 is a primary storage device implemented by using a random access memory (RAM) or the like. The storage device 1080 is a secondary storage device implemented by using a hard disk, a solid state drive (SSD), a memory card, read only memory (ROM), or the like.

The input/output interface 1100 is an interface connecting the computer 1000 and an input/output device. For example, an input apparatus such as a keyboard and an output apparatus such as a display apparatus are connected to the input/output interface 1100.

The network interface 1120 is an interface connecting the computer 1000 to a network. The network may be a local area network (LAN) or a wide area network (WAN).

The storage device 1080 stores a program (program for realizing the above-described application) for implementing each functional configuration unit of the map generation apparatus 2000. The processor 1040 implements each functional configuration unit of the map generation apparatus 2000 by reading and executing this program in the memory 1060.

The map generation apparatus 2000 may be implemented by a single computer 1000 or may be implemented by a plurality of computers 1000. In the latter case, the configurations of the computers 1000 do not need to be the same, and can be different from each other.

### <Flow of Processing>

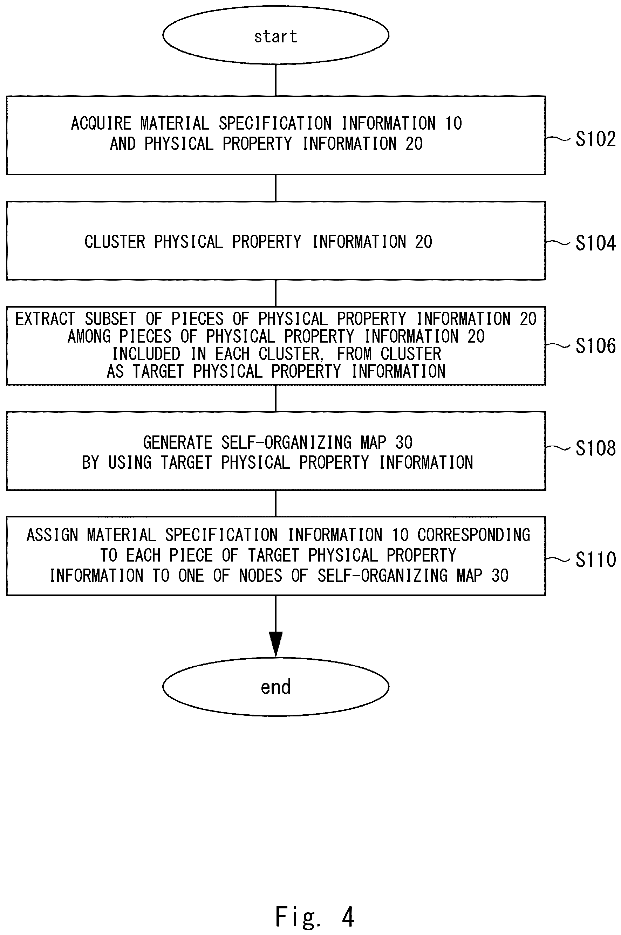

Fig. 4 is a flowchart illustrating a flow of processing executed by the map generation apparatus 2000 in the first example embodiment. The acquisition unit 2020 acquires material specification information 10 and physical property information 20 for each of the plurality of patterns of the material 60 (S102). The clustering execution unit 2040 performs clustering on the physical property information 20 to generate a plurality of clusters (S104). The generation unit 2060 extracts target physical property information from each cluster (S106). The generation unit 2060 generates a self-organizing map 30 by using the target physical property information (S108). The assignment unit 2080 assigns the material specification information 10 corresponding to the target physical property information to one of the nodes of the self-organizing map 30 (S110).

### <Acquisition of Material Specification Information 10 and Physical Property Information 20: S102>

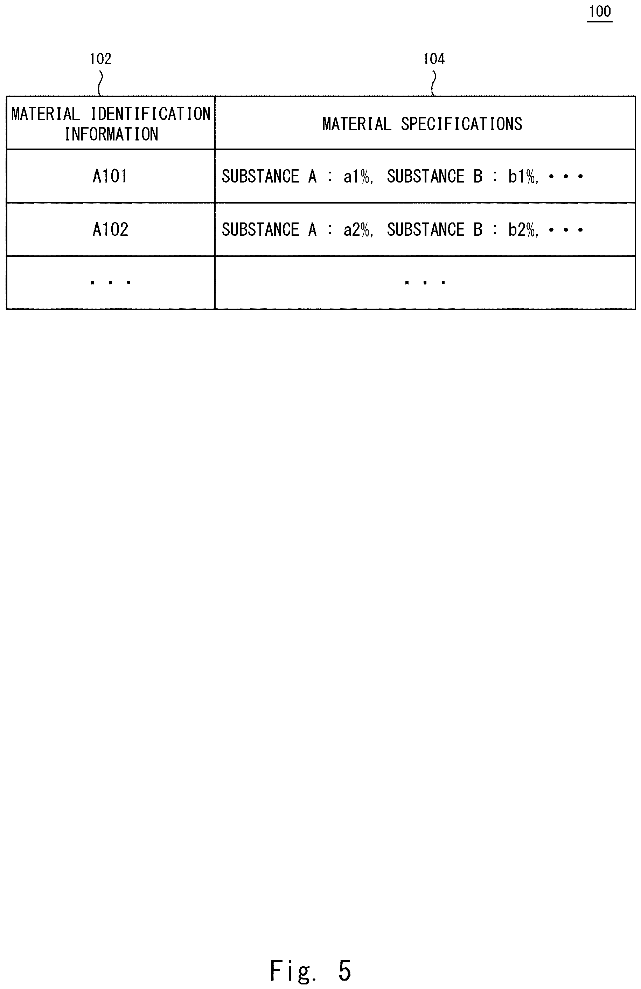

The acquisition unit 2020 acquires, for each of the plurality of patterns of the material 60 that can be used in the target step, the material specification information 10 indicating the material specification of the material 60 and the physical property information 20 of the product 70 that can be generated by using the material 60 (S102). Fig. 5 is a diagram illustrating the material specification information 10 in a table format. The table 100 of Fig. 5 has columns of material identification information 102 and material specification 104. The material identification information 102 indicates identification information assigned to the material 60. The material specification 104 indicates the specification of the material 60.

In Fig. 5, the material specification information 10 is represented by a record of the table 100. That is, the material specification information 10 associates the identification information of the material 60 with the material specification of the material 60 having that identification information.

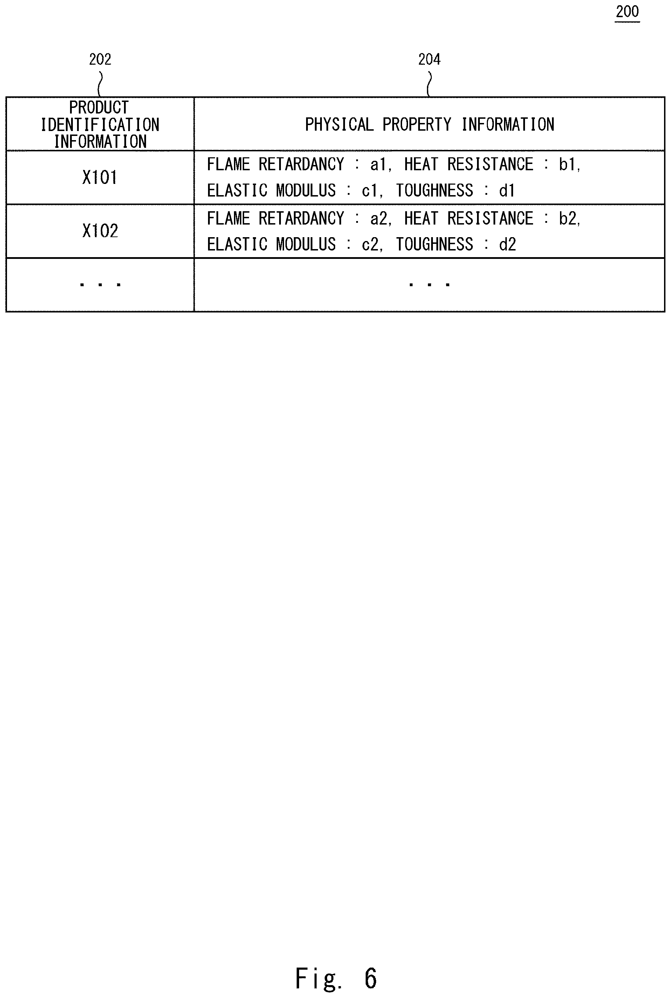

Fig. 6 is a diagram illustrating the physical property information 20 in a table format. The table 200 of Fig. 6 has columns of product identification information 202 and physical properties 204. The product identification information 202 indicates identification information of the product 70. The physical properties 204 indicates the physical properties of the product 70. In the table 200, the physical properties of the product 70 are represented by indicating an association of "a label representing the type of physical property: a physical property value of the physical property" for each physical property.

In Fig. 6, the physical property information 20 is represented by a record of the table 200. That is, the physical property information 20 associates the identification information of the product 70 with the physical property of the product 70 having that identification information.

The acquisition unit 2020 acquires a plurality of pairs of the material specification information 10 and the physical property information 20. There are various methods for the acquisition unit 2020 to acquire a pair of the material specification information 10 and the physical property information 20. For example, a pair of the material specification information 10 and the physical property information 20 is stored in advance in any storage device accessible from the map generation apparatus 2000. The acquisition unit 2020 accesses the storage device to acquire a pair of the material specification information 10 and the physical property information 20. **In** addition, for example, the acquisition unit 2020 may acquire a pair of the material specification information 10 and the physical property information 20 by receiving a user input for inputting a pair of the material specification information 10 and the physical property information 20. In addition, for example, the acquisition unit 2020 may acquire a pair of the material specification information 10 and the physical property information 20 by receiving a pair of the material specification information 10 and the physical property information 20 transmitted from another device.

Here, there are various methods of generating a pair of the material specification information 10 and the physical property information 20. For example, a pair of the material specification information 10 and the physical property information 20 is generated by simulating the generation of the product 70. Specifically, the physical property information 20 indicating the predicted value of the physical property value of each physical property is generated for the product 70 by performing simulation with specific material specification as an input. Then, a pair of the generated physical property information 20 and the material specification information 10 indicating the material specification given as the input is obtained. Here, a well-known technique can be used as a technique for acquiring material specification as an input and achieving a simulation for outputting prediction data of physical properties of a product generated in a specific step by using the material specified by the material specification.

In addition, for example, a pair of the material specification information 10 and the physical property information 20 may be generated by actually generating the product 70. Specifically, the product 70 is experimentally generated by using the material 60 represented by specific material specification in the target step. Furthermore, the physical property information 20 is generated by measuring the physical property value of each physical property for the generated product 70. As a result, a pair of the generated physical property information 20 and the material specification information 10 indicating the used material 60 is obtained.

Note that the physical property information 20 acquired by the acquisition unit 2020 may include physical property information with different data representation methods. For example, it is considered that different labels are used for physical properties that are essentially equivalent to each other. In addition, it is considered that physical property values of the same physical properties are represented by units different from each other. In such a case, the acquisition unit 2020 preferably unifies the data representation method by unifying labels, performing unit conversion, and the like. As described above, it is considered that the situation in which pieces of the physical property information 20 represent the data differently from each other may occur, for example, when both the physical property information 20 generated by using the simulation and the physical property information 20 generated by actually generating the product 70 are acquired. Note that it is preferable that such the unification of the data representation methods is performed for the material specification information 10.

### <Clustering of Physical Property Information 20: S104>

The clustering execution unit 2040 clusters the physical property information 20 (S104). Specifically, the clustering execution unit 2040 performs clustering of the physical property information 20 by clustering the physical property vectors obtained from each of the plurality of pieces of physical property information 20 acquired by the acquisition unit 2020.

For example, the clustering execution unit 2040 performs clustering of physical property vectors by using various clustering algorithms such as the k-means method. At this time, the clustering execution unit 2040 preferably performs dimensionality reduction on the physical property vector, and performs clustering by using the physical property vector on which the dimensionality reduction has been performed. As described above, by performing dimensionality reduction, it is possible to prevent an occurrence of a situation in which, in a case where a plurality of physical properties having high correlation is included in the physical property information 20, the self-organizing map 30 is strongly affected by these physical properties. Note that, as a method of dimensionality reduction, various methods such as a method using a neural network or a method using principal component analysis can be used.

As described above, the physical property vector is n-dimensional data representing the magnitude of the physical property value of each of n types of physical properties. The physical property vector may directly indicate the physical property value of each of the n types of physical properties indicated by the physical property information 20, or may indicate a value obtained by converting each physical property value by a predetermined method (for example, normalization, standardization, and the like).

Here, the number of physical properties indicated by the physical property information 20 may be more than n. In this case, a subset of the data indicated by the physical property information 20 is represented by the physical property vector. Here, which type of physical property among the physical properties indicated by the physical property information 20 is represented by a physical property vector (in other words, what type of physical properties are used for generating the self-organizing map 30) may be determined in advance or may be designated by the user.

### <Extraction of Target Physical Property Information: S106>

The generation unit 2060 extracts target physical property information from each cluster (S106). Here, there are various methods of extracting the target physical property information from the cluster. For example, the clustering execution unit 2040 randomly extracts a predetermined number of pieces of physical property information 20 from each cluster, and uses the extracted physical property information 20 as target physical property information.

The number of pieces of the target physical property information extracted from the cluster may be different for each cluster. For example, the clustering execution unit 2040 determines, for each cluster, the number of pieces of the target physical property information to be extracted from the cluster, based on the size of the cluster. Here, the larger the size of the cluster, the larger the number of pieces of the target physical property information to be extracted from the cluster.

For example, the clustering execution unit 2040 calculates a statistical value (for example, an average value) of the sizes of all clusters as a size reference value. For each cluster, the clustering execution unit 2040 calculates a ratio of the size of the cluster to a reference value, and multiplies the ratio by a predetermined reference number to obtain a value as the number of pieces of the target physical property information to be extracted from the cluster. It is assumed that any value is set in advance as the reference number.

Here, any method of determining the size of the cluster is adopted. For example, the size of the cluster is determined by the number of pieces of physical property information 20 included in the cluster. **In** addition, for example, the size of the cluster may be determined by the magnitude of variation in physical property vectors obtained from each piece of physical property information 20 included in the cluster.

### <Generation of Self-Organizing Map 30: S108>

The generation unit 2060 generates the self-organizing map 30 by using each piece of target physical property information (S108). The self-organizing map 30 has a plurality of nodes disposed on an m-dimensional map space (m=2 or m=3). Which one of two dimensions and three dimensions is adopted as the number of dimensions of the map space may be determined in advance or may be designated by the user. An n-dimensional physical property vector is assigned to each node of the self-organizing map 30.

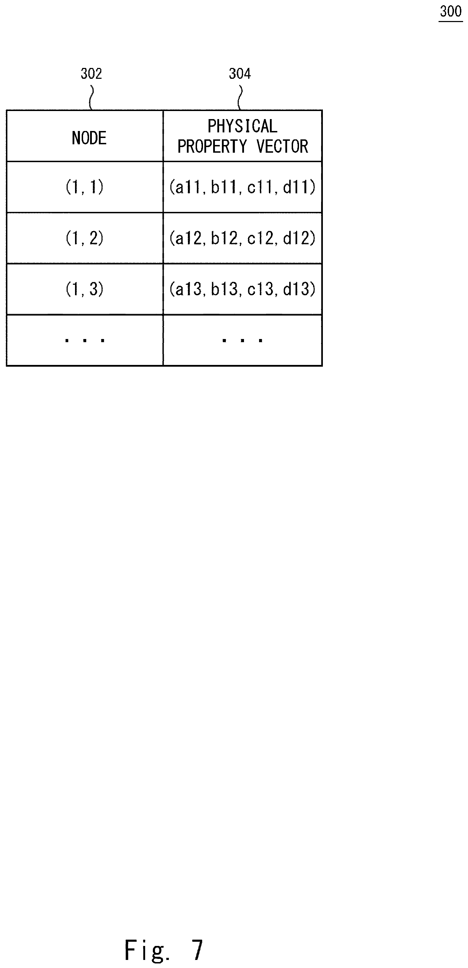

Fig. 7 is a diagram illustrating a structure of the self-organizing map 30 in a table format. The table 300 has two columns of a node 302 and a physical property vector 304. Each record of the table 300 indicates that the physical property vector indicated by the physical property vector 304 of the record is assigned to the node specified by the node 302 of the record. Note that, in Fig. 7, the node 302 indicates coordinates of a node on the map space.

The assignment of physical property vectors to each node is performed by training the self-organizing map 30. The training of the self-organizing map 30 can be performed by inputting n-dimensional training data used for training to the self-organizing map 30. Here, as a specific method of training the self-organizing map by using the training data, a well-known method can be used.

For example, the generation unit 2060 initializes the self-organizing map 30 by a certain method. As a method of initialization, for example, a method of initializing a physical property vector of each node to a random value can be adopted. The generation unit 2060 obtains a plurality of physical property vectors by obtaining a physical property vector from each of the plurality of pieces of target physical property information. The generation unit 2060 treats each of the plurality of physical property vectors as training data to train the self-organizing map 30, thereby generating the self-organizing map 30. As a result, the physical property vector corresponding to each node of the self-organizing map 30 becomes n-dimensional data indicating a value related to each of the physical property values of n types of physical properties.

### <Assignment of Material Specification Information 10: S110>

For each piece of target physical property information, the assignment unit 2080 assigns the material specification information 10 corresponding to the target physical property information to one of the nodes of the self-organizing map 30 (S110). Specifically, the assignment unit 2080 performs the following processes for each piece of target physical property information. First, the assignment unit 2080 determines a node having a physical property vector that is most similar to the physical property vector obtained from the target physical property information among the nodes of the self-organizing map 30. Then, the assignment unit 2080 assigns the material specification information 10 corresponding to the target physical property information to the determined node.

The degree of similarity between the physical property vectors can be determined, for example, based on a distance between the physical property vectors. Therefore, for example, the assignment unit 2080 calculates the distance of the physical property vector obtained from the target physical property information, from the physical property vector of each node of the self-organizing map 30. Then, the node having the smallest calculated distance is determined as the node having the physical property vector that is closest to the physical property vector obtained from the target physical property information.

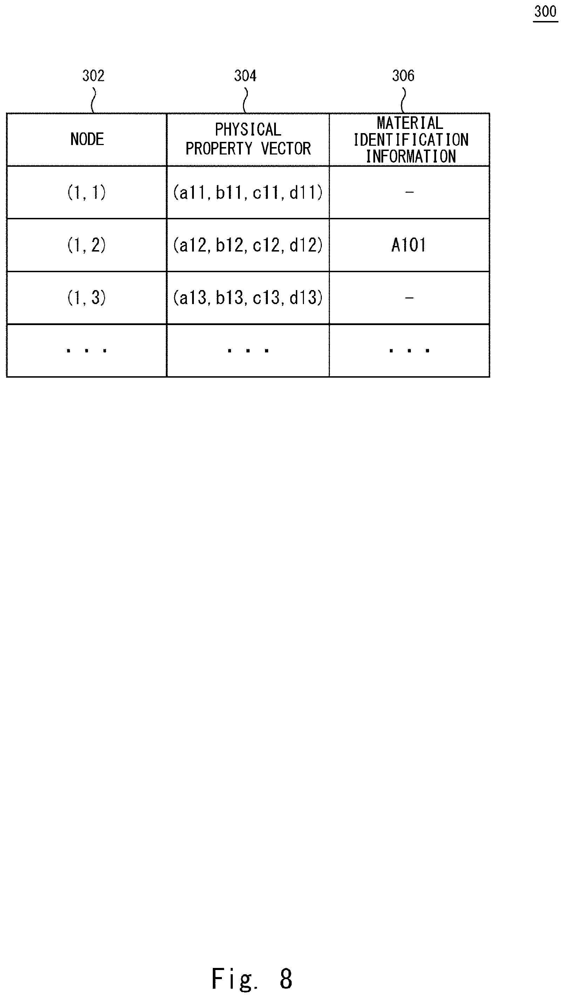

Fig. 8 is a diagram illustrating the structure of the self-organizing map 30 to which the material specification information 10 is assigned. The table 300 of Fig. 8 is different from the table 300 of Fig. 7 in that the table has a column of material identification information 306. The record indicating the node to which the material specification information 10 is assigned indicates the material specification information indicated by the material specification information 10 in the material identification information 306. On the other hand, in the record indicating the node to which the material specification information 10 is not assigned, the material identification information 306 is blank.

### <Output of Result>

The map generation apparatus 2000 outputs information representing the self-organizing map 30 to which the material specification information 10 has been assigned to the nodes (hereinafter, output information) in any form. Hereinafter, a functional configuration unit of the map generation apparatus 2000 that generates and outputs output information is referred to as an output unit.

For example, the output unit stores the output information in any storage device. In addition, for example, the output unit outputs the output information to a display device to cause the display device to display the output information. In addition, for example, the output unit transmits the output information to any other device.

### <Example of Output Information>

For example, the output information includes an image that visually represents the self-organizing map 30 (hereinafter, a map image). The map image is an image that represents a correspondence between the distribution of the material specifications and the distribution of the physical properties.

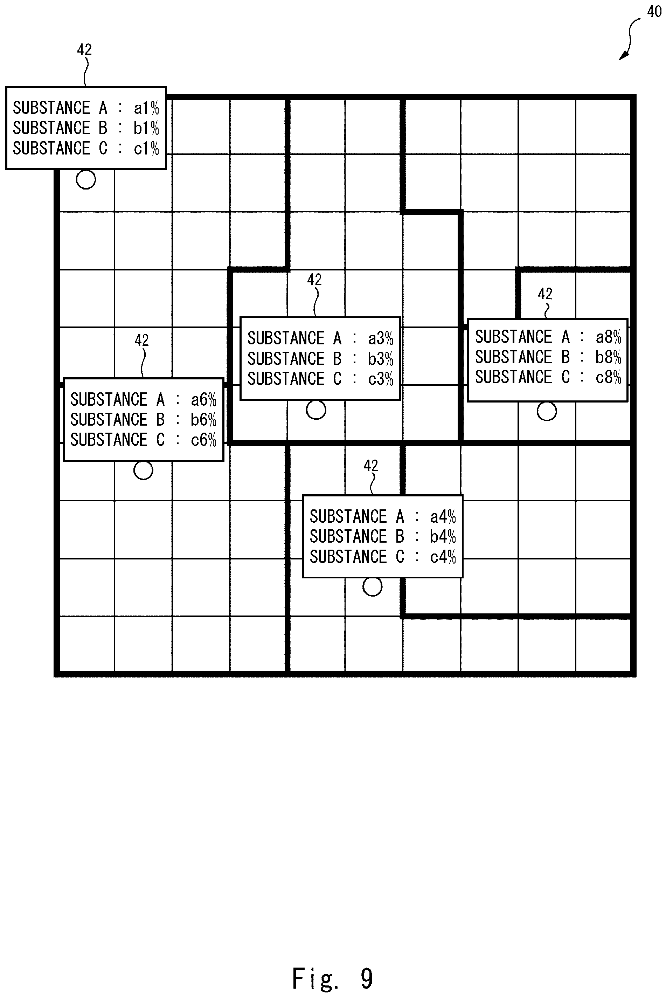

Fig. 9 is a diagram illustrating the map image. In Fig. 9, a map image 40 visually represents the map space of the self-organizing map 30. A material specification display 42 indicating a part or the entirety of the material specification information 10 is superimposed on the node to which the material specification information 10 is assigned.

Nodes of the map image 40 are divided into clusters based on the physical property vector. The thick frame of the map image 40 represents the boundary of the cluster. In order to divide the nodes into clusters, the map generation apparatus 2000 performs clustering on physical property vectors corresponding to the respective nodes of the self-organizing map 30. By dividing the physical property vector into clusters in this manner, it is possible to also divide nodes corresponding to the physical property vector into clusters. Note that the method of clustering the physical property vectors is as described above.

Each node of the map image 40 may be colored based on the physical property vector. Here, various existing methods can be used as a method of performing coloring on each node of the self-organizing map according to data associated with the node.

Although the present invention has been described above with reference to the example embodiment, the present invention is not limited to the above-described example embodiment. Various changes that can be understood by those skilled in the art can be made to the configuration and details of the present invention within the scope of the present invention.

In the above-described example, the program includes instructions (or software codes) that, when loaded into a computer, cause the computer to perform one or more of the functions described in the embodiments. The program may be stored in a non-transitory computer readable medium or a tangible storage medium. By way of example, and not a limitation, non-transitory computer readable media or tangible storage media can include a random-access memory (RAM), a read-only memory (ROM), a flash memory, a solid-state drive (SSD) or other types of memory technologies, a CD-ROM, a digital versatile disc (DVD), a Blu-ray disc or other types of optical disc storage, and magnetic cassettes, magnetic tape, magnetic disk storage or other types of magnetic storage devices. The program may be transmitted on a transitory computer readable medium or a communication medium. By way of example, and not a limitation, transitory computer readable media or communication media can include electrical, optical, acoustical, or other forms of propagated signals.

Some or all of the above-described example embodiments may be described as in the following Supplementary Notes, but are not limited to the following Supplementary Notes.

### (Supplementary Note 1)

A map generation apparatus comprising:
acquisition means for acquiring a plurality of pieces of material specification information indicating a material specification, and acquiring physical property information indicating a physical property value of each of a plurality of physical properties of a product that can be generated with the material specification indicated by the material specification information for each piece of the material specification information;
clustering execution means for clustering a plurality of pieces of the physical property information;
generation means for extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
assignment means for assigning the material specification information corresponding to the extracted physical property information to one of the nodes.

### (Supplementary Note 2)

The map generation apparatus according to supplementary note 1,
wherein the clustering execution means clusters the physical property information by clustering the physical property vector obtained from each of a plurality of pieces of the physical property information.

### (Supplementary Note 3)

The map generation apparatus according to supplementary note 2,
wherein the clustering execution means performs dimensionality reduction on the physical property vector obtained from the physical property information, and clusters the physical property vector after the dimensionality reduction is performed.

### (Supplementary Note 4)

The map generation apparatus according to any one of supplementary notes 1 to 3,
wherein the assignment means determines, for each piece of the extracted physical property information, the node associated with the physical property vector similar to the physical property vector obtained from that physical property information, and assigns the material specification information corresponding to that physical property information to the determined node.

### (Supplementary Note 5)

The map generation apparatus according to any one of supplementary notes 1 to 3,
wherein the physical property information corresponding to the material specification information indicates information regarding a physical property of a product that can be generated by using, as a material in a second step after the first step, a product that can be generated in a first step by using material specification indicated by that material specification information.

### (Supplementary Note 6)

The map generation apparatus according to any one of supplementary notes 1 to 3, further comprising output means for outputting an image representing each node of the self-organizing map and information regarding the material specification indicated by the material specification information associated with the node.

### (Supplementary Note 7)

A map generation method executed by a computer, the method comprising:
an acquisition step of acquiring a plurality of pieces of material specification information indicating a material specification, and acquiring physical property information indicating a physical property value of each of a plurality of physical properties of a product that can be generated with the material specification indicated by the material specification information for each piece of the material specification information;
a clustering execution step of clustering a plurality of pieces of the physical property information;
a generation step of extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
an assignment step of assigning the material specification information corresponding to the extracted physical property information to one of the nodes.

### (Supplementary Note 8)

The map generation method according to supplementary note 7,
wherein, in the clustering execution step, clustering the physical property information by clustering the physical property vector obtained from each of a plurality of pieces of the physical property information.

### (Supplementary Note 9)

The map generation method according to supplementary note 8,
wherein, in the clustering execution step, performing dimensionality reduction on the physical property vector obtained from the physical property information, and clustering the physical property vector after the dimensionality reduction is performed.

### (Supplementary Note 10)

The map generation method according to any one of supplementary notes 7 to 9,
wherein, in the assignment step, determining, for each piece of the extracted physical property information, the node associated with the physical property vector similar to the physical property vector obtained from that physical property information, and assigning the material specification information corresponding to that physical property information to the determined node.

### (Supplementary Note 11)

The map generation method according to any one of supplementary notes 7 to 9,
wherein the physical property information corresponding to the material specification information indicates information regarding a physical property of a product that can be generated by using, as a material in a second step after the first step, a product that can be generated in a first step by using material specification indicated by that material specification information.

### (Supplementary Note 12)

The map generation method according to any one of supplementary notes 7 to 9, further comprising an output unit configured to output an image representing each node of the self-organizing map and information regarding the material specification indicated by the material specification information associated with the node.

### (Supplementary Note 13)

A non-transitory computer readable medium storing a program for causing a computer to execute:
an acquisition step of acquiring a plurality of pieces of material specification information indicating a material specification, and acquiring physical property information indicating a physical property value of each of a plurality of physical properties of a product that can be generated with the material specification indicated by the material specification information for each piece of the material specification information;
a clustering execution step of clustering a plurality of pieces of the physical property information;
a generation step of extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
an assignment step of assigning the material specification information corresponding to the extracted physical property information to one of the nodes.

### (Supplementary Note 14)

The computer readable medium according to supplementary note 13,
wherein, in the clustering execution step, clustering the physical property information by clustering the physical property vector obtained from each of a plurality of pieces of the physical property information.

### (Supplementary Note 15)

The computer readable medium according to supplementary note 14,
wherein, in the clustering execution step, performing dimensionality reduction on the physical property vector obtained from the physical property information, and clustering the physical property vector after the dimensionality reduction is performed is clustered.

### (Supplementary Note 16)

The computer readable medium according to any one of supplementary notes 13 to 15,
wherein, in the assignment step, determining, for each piece of the extracted physical property information, the node associated with the physical property vector similar to the physical property vector obtained from that physical property information, and assigning the material specification information corresponding to that physical property information to the determined node.

### (Supplementary Note 17)

The computer readable medium according to any one of supplementary notes 13 to 15,
wherein the physical property information corresponding to the material specification information indicates information regarding a physical property of a product that can be generated by using, as a material in a second step after the first step, a product that can be generated in a first step by using material specification indicated by that material specification information.

### (Supplementary Note 18)

The computer readable medium according to any one of supplementary notes 13 to 15, further comprising an output unit configured to output an image representing each node of the self-organizing map and information regarding material specification indicated by the material specification information associated with the node.

### Reference Signs List

- 10: MATERIAL SPECIFICATION INFORMATION
- 20: PHYSICAL PROPERTY INFORMATION
- 30: SELF-ORGANIZING MAP
- 40: MAP IMAGE
- 42: MATERIAL SPECIFICATION DISPLAY
- 60: MATERIAL
- 70: PRODUCT
- 100: TABLE
- 102: MATERIAL IDENTIFICATION INFORMATION
- 104: MATERIAL SPECIFICATION
- 200: TABLE
- 202: PRODUCT IDENTIFICATION INFORMATION
- 204: PHYSICAL PROPERTIES
- 300: TABLE
- 302: NODE
- 304: PHYSICAL PROPERTY VECTOR
- 306: MATERIAL IDENTIFICATION INFORMATION
- 1000: COMPUTER
- 1020: BUS
- 1040: PROCESSOR
- 1060: MEMORY
- 1080: STORAGE DEVICE
- 1100: INPUT/OUTPUT INTERFACE
- 1120: NETWORK INTERFACE
- 2000: MAP GENERATION APPARATUS
- 2020: ACQUISITION UNIT
- 2040: CLUSTERING EXECUTION UNIT
- 2060: GENERATION UNIT
- 2080: ASSIGNMENT UNIT

## Claims

1. A map generation apparatus comprising:
acquisition means for acquiring a plurality of pieces of material specification information indicating a material specification, and acquiring physical property information indicating a physical property value of each of a plurality of physical properties of a product that can be generated with the material specification indicated by the material specification information for each piece of the material specification information;
clustering execution means for clustering a plurality of pieces of the physical property information;
generation means for extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
assignment means for assigning the material specification information corresponding to the extracted physical property information to one of the nodes.

2. The map generation apparatus according to claim 1,
wherein the clustering execution means clusters the physical property information by clustering the physical property vector obtained from each of a plurality of pieces of the physical property information.

3. The map generation apparatus according to claim 2,
wherein the clustering execution means performs dimensionality reduction on the physical property vector obtained from the physical property information, and clusters the physical property vector after the dimensionality reduction is performed.

4. The map generation apparatus according to any one of claims 1 to 3,
wherein the assignment means determines, for each piece of the extracted physical property information, the node associated with the physical property vector similar to the physical property vector obtained from that physical property information, and assigns the material specification information corresponding to that physical property information to the determined node.

5. The map generation apparatus according to any one of claims 1 to 3,
wherein the physical property information corresponding to the material specification information indicates information regarding a physical property of a product that can be generated by using, as a material in a second step after the first step, a product that can be generated in a first step by using material specification indicated by that material specification information.

6. The map generation apparatus according to any one of claims 1 to 3, further comprising output means for outputting an image representing each node of the self-organizing map and information regarding the material specification indicated by the material specification information associated with the node.

7. A map generation method executed by a computer, the method comprising:
an acquisition step of acquiring a plurality of pieces of material specification information indicating a material specification, and acquiring physical property information indicating a physical property value of each of a plurality of physical properties of a product that can be generated with the material specification indicated by the material specification information for each piece of the material specification information;
a clustering execution step of clustering a plurality of pieces of the physical property information;
a generation step of extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
an assignment step of assigning the material specification information corresponding to the extracted physical property information to one of the nodes.

8. The map generation method according to claim 7,
wherein, in the clustering execution step, clustering the physical property information by clustering the physical property vector obtained from each of a plurality of pieces of the physical property information.

9. The map generation method according to claim 8,
wherein, in the clustering execution step, performing dimensionality reduction on the physical property vector obtained from the physical property information, and clustering the physical property vector after the dimensionality reduction is performed.

10. The map generation method according to any one of claims 7 to 9,
wherein, in the assignment step, determining, for each piece of the extracted physical property information, the node associated with the physical property vector similar to the physical property vector obtained from that physical property information, and assigning the material specification information corresponding to that physical property information to the determined node.

11. The map generation method according to any one of claims 7 to 9,
wherein the physical property information corresponding to the material specification information indicates information regarding a physical property of a product that can be generated by using, as a material in a second step after the first step, a product that can be generated in a first step by using material specification indicated by that material specification information.

12. The map generation method according to any one of claims 7 to 9, further comprising an output unit configured to output an image representing each node of the self-organizing map and information regarding the material specification indicated by the material specification information associated with the node.

13. A non-transitory computer readable medium storing a program for causing a computer to execute:
an acquisition step of acquiring a plurality of pieces of material specification information indicating a material specification, and acquiring physical property information indicating a physical property value of each of a plurality of physical properties of a product that can be generated with the material specification indicated by the material specification information for each piece of the material specification information;
a clustering execution step of clustering a plurality of pieces of the physical property information;
a generation step of extracting, from each of a plurality of clusters generated by the clustering, a subset of pieces of the physical property information among a plurality of pieces of the physical property information included in the cluster, and generating, by using the extracted physical property information, a self-organizing map in which a physical property vector indicating a value related to the physical property value of each of the plurality of types of physical properties of the product is assigned to each node on a map space; and
an assignment step of assigning the material specification information corresponding to the extracted physical property information to one of the nodes.

14. The computer readable medium according to claim 13,
wherein, in the clustering execution step, clustering the physical property information by clustering the physical property vector obtained from each of a plurality of pieces of the physical property information.

15. The computer readable medium according to claim 14,
wherein, in the clustering execution step, performing dimensionality reduction on the physical property vector obtained from the physical property information, and clustering the physical property vector after the dimensionality reduction is performed is clustered.

16. The computer readable medium according to any one of claims 13 to 15,
wherein, in the assignment step, determining, for each piece of the extracted physical property information, the node associated with the physical property vector similar to the physical property vector obtained from that physical property information, and assigning the material specification information corresponding to that physical property information to the determined node.

17. The computer readable medium according to any one of claims 13 to 15,
wherein the physical property information corresponding to the material specification information indicates information regarding a physical property of a product that can be generated by using, as a material in a second step after the first step, a product that can be generated in a first step by using material specification indicated by that material specification information.

18. The computer readable medium according to any one of claims 13 to 15, further comprising an output unit configured to output an image representing each node of the self-organizing map and information regarding material specification indicated by the material specification information associated with the node.
